# EUROPÄISCHE PATENTANMELDUNG

(11) **EP 0 537 132 A2**
(43) Veröffentlichungstag der Anmeldung: **14.04.1993**
(21) Anmeldenummer: 92890082.8
(22) Anmeldetag: 06.04.1992
(51) Int. Cl.: G01N 35/08, G01N 33/00

(54) **Analysengerät**

(30) Priorität: 09.10.1991 AT 2005/91
(71) Anmelder: AVL Medical Instruments AG, CH-8207 Schaffhausen (CH)
(72) Erfinder: Rüther, Horst, Dipl.-Ing. Dr., A-8303 Gritsch Nr. 19 (AT)
(74) Vertreter: Krause, Walter, Dr. Dipl.-Ing.

(57) **Zusammenfassung**

Ein Analysengerät, insbesondere zur Untersuchung von Körperflüssigkeiten, mit einem Meßmodul (1), zumindest einer Gaszuleitung (13) für ein Kalibriergas, einer Befeuchtungseinrichtung (12) für das Kalbibriergas sowie einer mit dem Meßkanal (2) des Meßmoduls verbindbaren Waschwasserleitung (6), wird dadurch vereinfacht und verbessert daß die Befeuchtungseinrichtung (12) für das Kalibriergas über einen Wasserzu- (10) und einen Wasserablauf (11) in die Waschwasserleitung (6) integriert ist. Das Kalibriergas kann über die Wasseroberfläche (16) in der Befeuchtungseinrichtung (12) geleitet und so angefeuchtet werden.

## Beschreibung

Die Erfindung betrifft ein Analysengerät, insbesondere zur Untersuchung von Körperflüssigkeiten, mit einem Meßmodul, zumindest einer Gaszuleitung für ein Kalibriergas, einer Befeuchtungseinrichtung für das Kalbibriergas sowie einer mit dem Meßkanal des Meßmoduls verbindbaren Waschwasserleitung.

Ein derartiges Analysengerät ist beispielsweise aus der EPA 0 297 082 bekannt geworden. Neben einer Eingabeeinrichtung für Proben, kann der Meßkanal des Meßmoduls dieses Analysengerätes über ein Andockelement unter anderem mit mehreren Kalibriergasen und einer Waschwasserleitung verbunden werden. In vorgegebenen bzw. vom Benützer festgelegten Intervallen wird das Meßmodul von einer Reinigungsflüssigkeit, z.B. Wasser, aus einem Wasserreservoir des Gerätes durchströmt. Zur Befeuchtung der Kalibriergase werden diese von unten in einen mit Wasser gefüllten Behälter einer Befeuchtungseinrichtung eingeleitet und durch das Wasser hindurchperlen gelassen. Der Behälter der Befeuchtungseinrichtung weist ventilgeschaltete Versorgungsanschlüsse zum Wasserreservoir und zu einem Abfallbehälter auf, über welche das Wasser in der Befeuchtungseinrichtung in vorgegebenen Zeitintervallen automatisch erneuert wird. Nachteilig bei diesem bekannten Analysengerät ist der relativ große Aufwand an Steuer- und Überwachungseinrichtungen, welche für die Wascheinrichtung einerseits und die Befeuchtungseinrichtung andererseits notwendig sind.

Aufgabe dieser Erfindung ist die Realisierung eines einfachen, kostengünstigen Analysengerätes, welches für die Funktionen Befeuchtung und Reinigung mit möglichst wenigen aktiv zu steuernden Elementen auskommt.

Diese Aufgabe wird erfindungsgemäß dadurch gelöst, daß die Befeuchtungseinrichtung für das Kalibriergas über einen Wasserzu- und einen Wasserablauf in die Waschwasserleitung integriert ist. Mit dieser Maßnahme wird das Gerät wesentlich vereinfacht, da separate Leitungen bzw. Ventile für die Befeuchtungseinrichtung entfallen. Bei jedem Waschvorgang des Meßmoduls wird Wasser aus dem Reservoir des Gerätes entnommen und gelangt durch die Befeuchtungseinrichtung in den Meßkanal des Meßmoduls, wodurch gleichzeitig die Wasserfüllung der Befeuchtungseinrichtung in regelmäßigen Intervallen erneuert wird.

Üblicherweise werden Gase derart befeuchtet, daß sie durch ein Wasserreservoir hindurchperlen, wie das beispielsweise aus der eingangs erwähnten EPA 0 297 082 bekannt ist. Ein Nachteil dieser bekannten Methode tritt dann auf, wenn verschiedene Kalibriergase bzw. Gasgemische benötigt werden. Entweder ist eine relativ große Zeitspanne für die Umtonometrierung des Wassers in Kauf zu nehmen oder es ist für jedes Gasgemisch ein eigener Befeuchter vorzusehen. Unter Umtonometrierung versteht man dabei die Maßnahme, ein bestimmtes, in Wasser gelöstes Gas durch ein anderes Gas zu ersetzen.

Ein weiteres Ziel der Erfindung ist es nun, die Zeitspanne für die Bereitstellung angefeuchteter Gase auch bei Verwendung nur einer Befeuchtungseinrichtung zu verkürzen. Dieses Ziel wird erfindungsgemäß dadurch erreicht, daß die Einleitung des Kalibriergases in die Befeuchtungseinrichtung in Höhe des durch den Wasserablauf definierten Wasserpegels erfolgt, wobei das Kalibriergas nach dessen Einleitung über die Wasseroberfläche streicht. Wird nämlich der Gasfluß über die Wasseroberfläche geleitet, lassen sich problemlos Befeuchtungsgrade > 90° realisieren. Gleichzeitig läßt sich die Zeitspanne für die Bereitstellung verschiedener befeuchteter Gase um ein Vielfaches reduzieren, da die absolute Wassermenge sehr gering gehalten werden kann und keine komplette Umtonometrierung notwendig ist, da die im Wasser gelösten Restgase im Gegensatz zur bekannten Methode nur einen geringen Einfluß auf das jeweils über die Wasseroberfläche streichende Kalibriergas ausüben.

In einer Weiterbildung der Erfindung ist vorgesehen, daß der Wasserzu- und der Wasserablauf der Befeuchtungseinrichtung in gleicher Höhe angeordnet sind und daß die Einleitung des Kalibriergases über den Wasserzulauf erfolgt, wodurch ein besonders einfacher Aufbau der Befeuchtungseinrichtung gewährleistet ist.

Um zu verhindern, daß das aus der Befeuchtungseinrichtung abströmende Gas Wassertröpfchen in die Gasleitung mitreißt, weist die Befeuchtungseinrichtung erfindungsgemäß eine über dem Wasserpegel und unterhalb einer Öffnung zur Ableitung des angefeuchteten Kalibriergases angeordnete gas- und wasserdampfdurchlässige Sperrvorrichtung auf. Vorteilhafterweise kann diese Sperrvorrichtung eine gas- und wasserdampfdurchlässige Membran sein. Weiters kann die Sperrvorrichtung auch aus einem Plättchen mit zumindest einer kleinen Bohrung bestehen.

Schließlich ist erfindungsgemäß vorgesehen, daß die Sperrvorrichtung eine Kammer zum Abscheiden von Wassertropfen aufweist, welche in die Öffnung zur Ableitung des angefeuchteten Kalibriergases mündet und zur Wasseroberfläche eine Bohrung aufweist, wobei vorteilhafterweise die Kammer von zwei Bauteilen begrenzt ist, und sich deren Innenraum ausgehend von der Öffnung zur Ableitung des Kalibriergases sowie der Bohrung zur Wasseroberfläche trichterförmig erweitert.

Der Aufbau der Befeuchtungseinrichtung richtet sich nach den Gasflußmengen, die befeuchtet werden sollen. Für eine Gasflußmenge von ca. 5 ml/min reicht beispielsweise ein runder Behälter mit einem Innenvolumen von ca. 0,5 ml. Der Abstand des Wasserablaufes vom Boden des Befeuchters wird so gewählt, daß beim Abfließen des Wassers aufgrund der Oberflächenspannung eine ausreichende Restmenge zurückbleibt.

Die Stabilisierung der Gaskonzentration auf 0,1 % des Sollwertes ist mit der erfindungsgemäßen Methode bei einem Gasfluß von 5 ml/min in weniger als 10 Sekunden möglich. Bei Verwendung einer herkömmlichen Befeuchtungseinrichtung nimmt hingegen die Stabilisierung mehrere Minuten in Anspruch.

Die Erfindung wird im folgenden anhand von Zeichnungen näher erläutert. Es zeigen:
Fig. 1 ein Analysengerät mit einer Befeuchtungseinrichtung nach der Erfindung,
Fig. 2 die Befeuchtungseinrichtung im Detail,
Fig. 3 einen Schnitt entlang der Linie III-III in Fig. 2, sowie
Fig. 4 eine Ausführungsvariante nach Fig. 3.

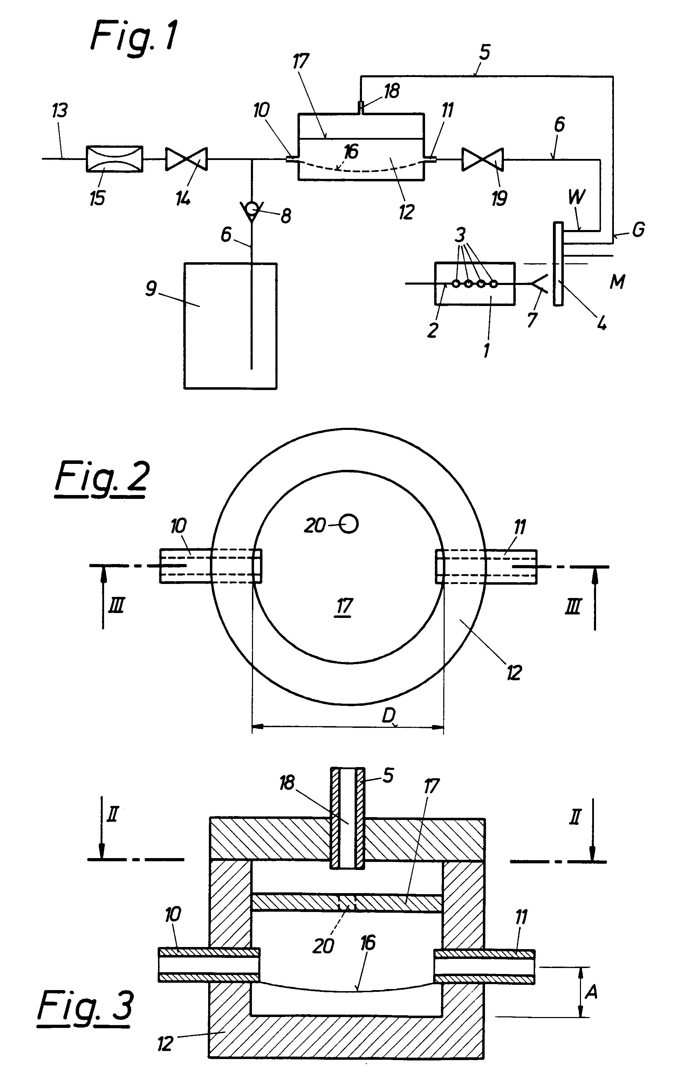

Von dem in Fig. 1 dargestellten Analysengerät sind nur die im Zusammenhang mit der vorliegenden Erfindung wesentlichen Teile dargestellt. So weist das Analysengerät ein Meßmodul 1 mit in einem Meßkanal 2 angeordneten Sensoren 3 auf, welches über ein bereits aus der eingangs zitierten EPA 0 297 082 bekanntes Andockelement 4 mit verschiedenen Zuleitungen verbunden werden kann. Es sei hier nur der mit der Gasleitung 5 verbundene Gasanschluß G zur Zufuhr der Kalibriergase, der mit der Waschwasserleitung 6 verbundene Wasseranschluß W sowie der Anschluß M zur Eingabe einer Probe aus einer Kapilare erwähnt. Die Probe kann auch direkt in den Einfüllstutzen 7 des Meßkanals 2 eingegeben werden, welcher mit dem Andockelement 4 zusammenwirkt. In die über ein Rückschlagventil 8 aus einem Wasserreservoir 9 geführte Waschwasserleitung 6 ist über einen Wasserzulauf 10 und einen Wasserablauf 11 eine Befeuchtungseinrichtung 12 integriert.

Über die Leitung 13 erfolgt die Einspeisung des bzw. der zu befeuchtenden Kalibriergase bzw. Kalibriergasgemische aus hier nicht weiter dargestellten Gasquellen. Die Einspeisung erfolgt vorteilhafterweise über eine dem Ventil 14 vorgeschaltete Blende 15 zur Stabilisierung des Gasflusses, wobei das Totvolumen zwischen Ventil 14 und Blende 15 gering gehalten werden soll, um starke Druckimpulse auf die Befeuchtungseinrichtung 12 zu vermeiden.

In einer nicht dargestellten Ausführungsvariante besteht auch die Möglichkeit die Blende 15 dem Ventil 14 nachzuschalten. Es müssen dann allerdings Vorkehrungen getroffen werden, die sicherstellen, daß kein Wasser zur Blende 15 gelangen kann. Das könnte beispielsweise durch den Einbau einer gasdurchlässigen jedoch wasserundurchlässigen Membran erreicht werden.

Die Einleitung des Kalibriergases in die Befeuchtungseinrichtung 12 erfolgt im dargestellten Beispiel über die Wasserzuleitung 10, wobei das Kalibriergas über die Wasseroberfläche 16 streicht, angefeuchtet wird und die Befeuchtungseinrichtung 12 nach dem Passieren einer gas- und wasserdampfdurchlässigen Sperrvorrichtung 17 durch eine oberhalb der Sperrvorrichtung 17 angeordnete, mit der Gasleitung 5 verbundene Öffnung 18 verläßt.

Das Analysengerät benötigt für die Zufuhr angefeuchteter Kalibriergase sowie für den Waschvorgang im wesentlichen nur zwei steuerbare Ventile, nämlich das Ventil 14 und ein zwischen Befeuchtungseinrichtung 12 und Wasseranschluß W in der Waschwasserleitung 6 angeordnetes Ventil 19. Während des Waschvorganges ist das Ventil 14 geschlossen und das Ventil 19 offen, bei der Versorgung des Meßmoduls mit einem Kalibriergas erfolgt die Beschaltung der Ventile umgekehrt.

In den Fig. 2 und 3 ist eine mögliche Ausführungsvariante einer Befeuchtungseinrichtung 12 nach Fig. 1 dargestellt. Gleiche Teile sind mit gleichen Bezugszeichen versehen. Aufgrund der kleinen Abmessungen der beispielsweise als zylindrisches Gefäß ausgeführten Befeuchtungseinrichtung 12 mit einem Durchmesser D im Bereich von 10 mm, stellt sich in Abhängigkeit von der Höhe des Wasserablaufes 11 ein Wasserpegel mit der Höhe A von wenigen Millimetern ein, wobei die Wasseroberfläche durch die Oberflächenspannung des Wassers konkav gekrümmt ist. Die Befeuchtungseinrichtung 12 beinhaltet somit nur einige 100 µl Wasser. Die Sperrvorrichtung 17 kann auch - wie dargestellt - als scheibenförmiges Plättchen mit einer kleinen exzentrischen Bohrung 20 ausgeführt sein, wodurch das Mitreißen von Wassertröpfchen in die Gasleitung 5 verhindert wird.

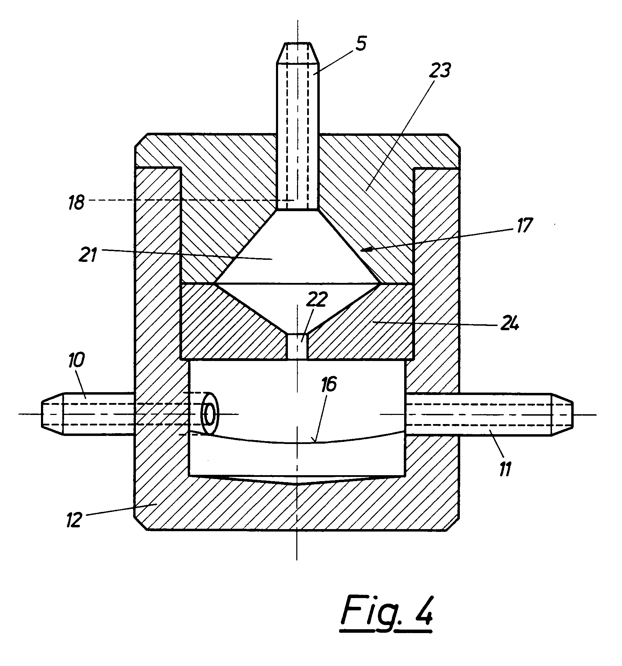

Bei der in Fig. 4 dargestellten Ausführungsvariante weist die Sperrvorrichtung 17 eine Kammer 21 zum Abscheiden von Wassertropfen auf. Die Kammer 21 wird von zwei Bauteilen 23 und 24 begrenzt, wobei in den Bauteil 23 die Gasleitung 5 eingeklebt ist und der Bauteil 24 eine Bohrung 22 zur Wasseroberfläche 16 aufweist. Der Innenraum der Kammer 21 erweitert sich ausgehend von der Öffnung 18 und der Bohrung 22 trichterförmig, wodurch allfällige Tropfen problemlos ablaufen können.

Im Rahmen der Erfindung ist es auch möglich, das zu befeuchtende Gas über mehrere am Umfang der Befeuchtungskammer in Höhe der Wasseroberfläche verteilte Öffnungen einzuleiten bzw. den Wasserzulauf im Boden der Befeuchtungseinrichtung anzuordnen.

## Patentansprüche

1. Analysengerät, insbesondere zur Untersuchung von Körperflüssigkeiten, mit einem Meßmodul, zumindest einer Gaszuleitung für ein Kalibriergas, einer Befeuchtungseinrichtung für das Kalbibriergas sowie einer mit dem Meßkanal des Meßmoduls verbindbaren Waschwasserleitung, **dadurch gekennzeichnet**, daß die Befeuchtungseinrichtung (12) für das Kalibriergas über einen Wasserzu- (10) und einen Wasserablauf (11) in die Waschwasserleitung (6) integriert ist.

2. Analysengerät nach Anspruch 1, **dadurch gekennzeichnet**, daß die Einleitung des Kalibriergases in die Befeuchtungseinrichtung (12) in Höhe des durch den Wasserablauf (11) definierten Wasserpegels erfolgt, wobei das Kalibriergas nach dessen Einleitung über die Wasseroberfläche (16) streicht.

3. Analysengerät nach Anspruch 2, **dadurch gekennzeichnet**, daß der Wasserzu- (10) und der Wasserablauf (11) der Befeuchtungseinrichtung (12) in gleicher Höhe angeordnet sind und daß die Einleitung des Kalibriergases über den Wasserzulauf (10) erfolgt.

4. Analysengerät nach Anspruch 2 oder 3, **dadurch gekennzeichnet**, daß die Befeuchtungseinrichtung (12) eine über dem Wasserpegel und unterhalb einer Öffnung (18) zur Ableitung des angefeuchteten Kalibriergases angeordnete gas- und wasserdampfdurchlässige Sperrvorrichtung (17) aufweist.

5. Analysengerät nach Anspruch 4, **dadurch gekennzeichnet**, daß die Sperrvorrichtung (17) eine gas- und wasserdampfdurchlässige Membran ist.

6. Analysengerät nach Anspruch 4, **dadurch gekennzeichnet**, daß die Sperrvorrichtung (17) eine Kammer (21) zum Abscheiden von Wassertropfen aufweist, welche in die Öffnung (18) zur Ableitung des angefeuchteten Kalibriergases mündet und zur Wasseroberfläche eine Bohrung (22) aufweist.

7. Analysengerät nach Anspruch 6, **dadurch gekennzeichnet**, daß die Kammer (21) von zwei Bauteilen (23, 24) begrenzt ist, und sich deren Innenraum ausgehend von der Öffnung (18) zur Ableitung des Kalibriergases sowie der Bohrung (22) zur Wasseroberfläche trichterförmig erweitert.
